# EUROPÄISCHE PATENTSCHRIFT

(11) **EP 0 407 653 B1**
(45) Veröffentlichungstag und Bekanntmachung des Hinweises auf die Patenterteilung: **14.12.1994**
(21) Anmeldenummer: 89117832.9
(22) Anmeldetag: 27.09.1989
(51) Int. Cl.: G01N 33/28

(54) **Verfahren zur Feststellung des Alkoholgehaltes und/oder des Heizwertes von Kraftstoffen**
Method for the determination of the alcohol content and/or the calorific value of fuels
Méthode de détermination de la teneur en alcool et/ou la valeur calorifique de carburants

(30) Priorität: 12.07.1989 DE 3922851
(43) Veröffentlichungstag der Anmeldung: 16.01.1991
(73) Patentinhaber: FEV Motorentechnik GmbH & Co. KG, D-52078 Aachen (DE)
(72) Erfinder: Schmitz, Günter, Dr.-Ing., D-5100 Aachen (DE); Kutz, Hans-Jürgen, Dipl.-Ing., D-5100 Aachen (DE); Reggelin, Bernd, Dipl.-Ing., D-5100 Aachen (DE)
(74) Vertreter: Langmaack, Jürgen, Dipl.-Ing.

(56) Entgegenhaltungen:
- US-A- 4 288 741
- IEEE TRANS. VEHICULAR TECHN., Band VT-27, Nr. 3, August 1978, IEEE, New York, NY (US); J.W. HILE et al., Seiten 142-144#

## Beschreibung

Die Erfindung bezieht sich auf ein Verfahren zur Feststellung des Alkoholgehaltes und/oder des Heizwertes von Kraftstoffen durch Messung elektrisch meßbarer Größen in einer den Kraftstoff enthaltenden Meßzelle zur Bestimmung der Dielektrizitätszahl des in der Zelle enthaltenen Gemisches als Kenngröße des Alkoholgehaltes bzw. des Heizwertes.

In Anbetracht der langfristig nur in begrenztem Umfang verfügbaren Reserven an fossiler Energie, insbesondere der aus Rohöl gewonnenen Kraftstoffe, und in Anbetracht der steigenden Anforderungen an den Umweltschutz, wird diesen Kraftstoffen in zunehmendem Maße Methyl- oder Äthylalkohol zugemischt. Dabei soll ein beliebiges Tanken sowohl von Reinkraftstoffen als auch von Mischkraftstoffen möglich sein. Bei höheren Alkoholanteilen ist dabei die Kenntnis des Mischungsverhältnisses erforderlich, um eine optimale Arbeitsweise der Brennkraftmaschine zu erreichen und dabei insbesondere eine genaue, den Betriebsverhältnissen angepaßte Kraftstoffzumessung zu ermöglichen. Besondere Probleme bereitet dabei die laufende Feststellung des Alkoholgehaltes des der Brennkraftmaschine im Betrieb laufend zugeführten Kraftstoffs bei Fahrzeugmotoren, bei denen durch das beliebige Tanken der Kraftstoffarten jede mögliche Mischung erreicht werden kann.

Die bekannten optischen Verfahren sind zu diesem Zweck kaum geeignet, da sie meist Grenzflächeneffekte zur Bestimmung des Brechungsindexes ausnutzen, aus dem dann auf den Alkoholanteil geschlossen werden kann. Abgesehen von der Schwierigkeit der Auswertung bei Fahrzeugmotoren ist ein Nachteil dieses Verfahrens auch, daß die messend zu beobachtende Mischung eine hohe Homogenität aufweisen muß, die insbesondere auch an der Grenzfläche vorhanden sein muß. Mit diesem Verfahren wurden nicht die erforderlichen Genauigkeiten erreicht, insbesondere auch durch Streuungen des Brechungsindexes der Grundkraftstoffe durch unterschiedliche Aromatengehalte.

Es wurde daher vorgeschlagen, den Alkoholanteil in Kraftstoffen durch eine Dielektrizitätsbestimmung festzustellen, und ein solches Verfahren hätte den Vorteil, daß die Problematik der Messung von Grenzflächeneffekten behoben ist, da die Messung volumetrisch erfolgt. Andererseits geht in die volumetrische Dielektrizitätsbestimmung in starkem Maße der Leitwert der Mischung ein (Querempfindlichkeit). Da aber der Leitwert sehr stark von Verunreinigungen oder Wasseranteilen abhängt, führt auch ein solches Meßverfahren häufig zu unbrauchbaren Ergebnissen. Eine Kombination beider Verfahren ist zu EP-0377782 A2 beschrieben.

In der Schrift "Proceedings of the Fourth International Symposium on Alcohol Fuels Technology", Sao Paulo, Brasilien, vom 05.10.1980, wird die Möglichkeit der Feststellung des Alkoholgehaltes von Kraftstoffen durch Dielektrizitätsmessungen beschrieben. Aufgrund der Einflüsse von Temperatur und Leitwert (hervorgerufen durch Wasseranteile oder andere Verschmutzungen im Kraftstoff) wurde das Verfahren jedoch verworfen, da eine für Verbrennungsmotoren geeignete, zuverlässige Messung nicht durchgeführt werden konnte.

Der Erfindung liegt daher die Aufgabe zugrunde, ein Verfahren der eingangs bezeichneten Art zu schaffen, das insbesondere in der Anwendung auf Fahrzeugmotoren eine genaue und zuverlässige Feststellung des Alkoholanteils an Kraftstoffen erlaubt.

Gemäß der Erfindung wird diese Aufgabe mit den im Anspruch 1 angegebenen Verfahrensschritten gelöst.

Hierdurch werden Störeinflüsse, welche die Kapazitätsmessung beeinträchtigen, verringert Vorteilhaft ist daß der Einfluß des Leitwertes des zu messenden Kraftstoffgemisches auf das Meßergebnis gering gehalten wird.

Dabei ist es besonders zweckmäßig, die Meßfrequenz in wenigstens zwei Stufen derart umzuschalten, daß sie in einem für den Einfluß die Kapazitätsmessung störender Größen unempfindlichen Bereich gehalten wird. Auch kann die Meßfrequenz durch eine Regelung auf einen für den Meßvorgang geeigneten Wert eingestellt und anschließend über den gesamten Bereich der Kraftstoffmischung konstant gehalten werden, wobei die bei der Regelung eingesetzte Stellgröße oder eine andere im Regelkreis meßbare Größe als Maß für den Alkoholgehalt bzw. den Heizwert ausgewertet wird.

Gemäß einem weiteren vorteilhaften Merkmal ist vorgesehen, daß wenigstens ein Kondensator, dessen Kapazitätswert bekannt ist, der zu messenden Kapazität zu- bzw. von ihr abgeschaltet wird. Auch kann die Meßfrequenz dadurch eingestellt bzw. angepaßt werden, daß eine Umschaltung zwischen Meßelektroden unterschiedlicher Kapazitätswerte erfolgt.

Besonders genaue Ergebnisse können dadurch erreicht werden, daß zusätzlich die Temperatur gemessen und zur Korrektur des festgestellten Kapazitätswertes verwendet wird. Auch ist es zweckmässig, daß der festgestellte Kapazitätswert durch Kombination mit einer schaltungstechnischen und/oder rechnerischen Kompensation korrigiert wird.

Bei Anwendung auf die Steuerung bzw. Regelung einer Einspritzbrennkraftmaschine ist gemäß einer weiteren bevorzugten Ausführungsform vorgesehen, daß die Messung des Alkoholgehaltes bzw. des Heizwertes des zugeführten Kraftstoffs zur Vorsteuerung der Einspritzmenge dient, während die Feinregulierung des Luftverhältnisses über eine Lambdaregelung bekannter Art erfolgt.

Ausführungsbeispiele der Erfindung werden anhand der Zeichnungen näher beschrieben.

Figur 1 zeigt eine Ausführungsform einer Meßzelle zur Durchführung des Verfahrens.

Figur 2 zeigt eine Ausführungsform einer Schaltung zur Durchführung des Verfahrens.

Figur 3 zeigt im Diagramm ein Ausführungsbeispiel der Frequenzanpassung bzw. Frequenzumschaltung bei der Durchführung des Verfahrens.

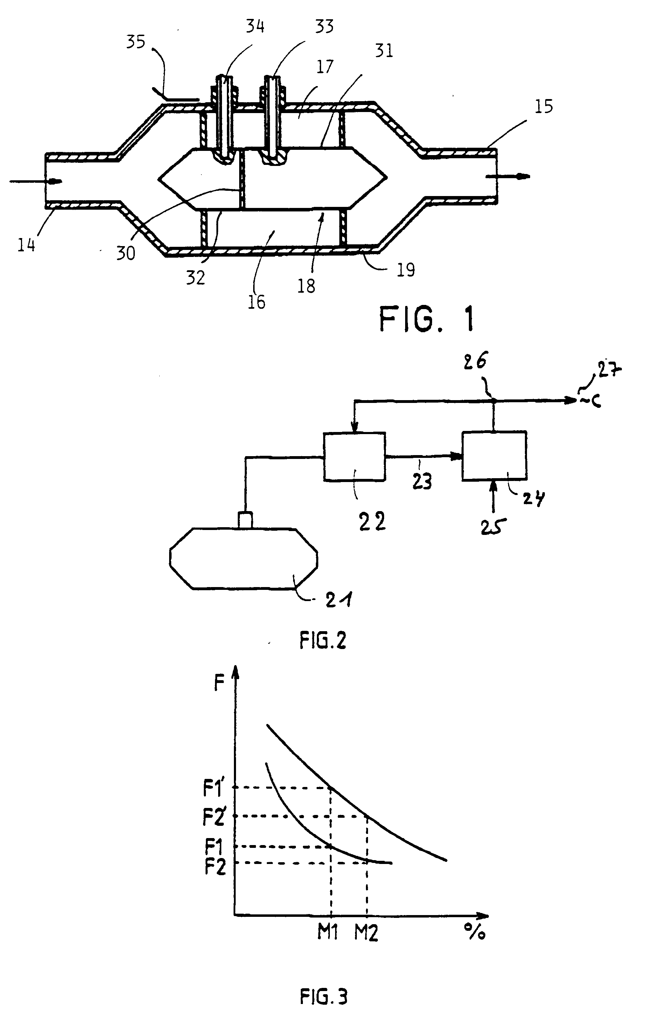

Figur 1 zeigt eine bevorzugte Ausführungsform einer Meßzelle zur Ausführung des Verfahrens gemäß der Erfindung. In die Meßzelle gelangt der Kraftstoff über Zufluß 14, und er verläßt die Meßzelle über Abfluß 15. Der Kraftstoffstrom teilt sich in der flächenhaften Darstellung der Fig. 1 auf in Strömungswege 16 und 17, die durch einen Mittelzylinder 18 gebildet werden. Wenn der Mittelzylinder 18 und der Außenmantel 19 ganz oder teilweise elektrisch leitend sind, können diese Wandungen bzw. Wandungsteile der Meßzelle die Elektroden des frequenzbestimmenden Kondensators einer Meß- bzw. Auswerteschaltung darstellen. Innerhalb der wenigstens teilweise leitfähigen Wandung der Meßzelle, die die erste Elektrode darstellt, ist dann der wenigstens teilweise leitfähige Mittelzylinder 18 als Strömungskörper die zweite Elektrode.

Die leitfähige Wandung des Mittelzylinders 18 ist durch eine geeignete Isolation 30 aufgeteilt in einen größeren Teil 31 und einen kleineren Teil 32. Abgriffe 33 und 34 der Teile 31 und 32 sind isoliert aus der Meßzelle herausgeführt.

Der Mittelzylinder 18 bildet mit seinen Teilen 31 und 32 zusammen mit dem Außenmantel 19 zwei Meßkondensatoren, die das Meßvolumen umschließen. Über Abgriff 33 kann dann gegenüber Anschluß 35 des Außenmantels 19 ein erster Wert abgegriffen werden, und dementsprechend kann über Abgriff 34 gegenüber Anschluß 35 ein weiterer Wert abgegriffen werden.

Es können die Werte sowohl für die Kapazität als auch für den Leitwert an denselben Abgriffen 33 bzw. 34 gegenüber Anschluß 35 abgegriffen werden. Diese Werte werden dann zur weiteren Verarbeitung in eine geeignete Schaltung eingegeben.

Durch die unterschiedlichen Abmessungen der beiden Teile 31 und 32 werden zwei Grundkapazitäten gebildet, die beispielsweise als frequenzbestimmendes Element einer schwingungsfähigen Schaltung eingesetzt werden können.

Besondere Vorteile lassen sich erreichen, wenn die Meßfrequenz durch eine Regelung konstant gehalten wird. Wie Fig. 2 zeigt, ist der Meßzelle 21 eine Meßschaltung 22 nachgeschaltet. Die von der Meßschaltung 22 in Abhängigkeit vom Alkoholgehalt des in Meßzelle 21 befindlichen Kraftstoffs erzeugte Frequenz auf Leitung 23 wird durch eine Schaltung 24 mit einem vorgegebenen Sollwert 25 verglichen. Aus diesem Vergleich resultiert im Punkt 26 eine Stellgröße, und diese beeinflußt die Meßschaltung 22 derart, daß bei einer auftretenden Differenz die Meßfrequenz an den Sollwert angeglichen wird. Es kann dabei beispielsweise die Stellgröße als Maß für den Alkoholgehalt an Klemme 27 ausgegeben werden.

Wenn die Kapazität einer in einer Meßzelle untergebrachten Kraftstoffmenge in einer Schaltung gemessen und ausgewertet wird, kann es zweckmäßig sein, daß die Schaltung schwingungsfähig ist. Ihre Ausgangsfrequenz wird als Maß der Kapazität ausgewertet, wobei die Kraftstoffmenge oder ein Teil davon das Dielektrikum des kapazitiven Teils der Schaltung bilden kann.

Da der Einfluß der Kapazität auf den Scheinwiderstand proportional mit der Frequenz ansteigt, der Einfluß des Leitwertes jedoch unabhängig von der Frequenz ist, scheint es zunächst sinnvoll, die Messungen mit einer möglichst hohen Frequenz durchzuführen. Dies bringt jedoch Probleme durch den Einfluß parasitärer Kapazitäten mit sich. Besondere Vorteile sind erreichbar, wenn die Frequenz zur Verringerung störender Einflüsse, z.B. durch den Leitwert, in umschaltbaren Bereichen gehalten werden kann. Diese Umschaltung kann z.B. durch Zu- oder Abschaltung von Kondensatoren bekannter Größe erfolgen, deren Einfluß auf die Meßfrequenz bekannt ist. Weitere Vorteile lassen sich erreichen, wenn die Anpassung der Meßfrequenz durch Umschaltung der Meßelektroden erfolgt, die z.B. durch unterschiedliche mechanische Abmessungen oder durch Unterschiede in ihrem Abstand zueinander verschiedene Grundkapazitäten bilden.

Wie Fig. 3 zeigt, kann die Umschaltung dabei jeweils mit einer Hysterese durchgeführt werden, um ein häufiges Umspringen im Grenzbereich zu vermeiden. Wird z.B. der Alkoholanteil M2 überschritten und sinkt infolgedessen die Meßfrequenz unter den Wert von F2, wird auf die höhere Meßfrequenz F2′ umgeschaltet. Dieser Meßbereich bleibt auch dann erhalten, wenn der Alkoholanteil wieder unter den Wert M2 absinkt. Erst beim Unterschreiten eines niedrigeren Alkoholanteils M1 und somit beim Überschreiten von FI′ wird wieder auf die niedrigere Meßfrequenz FI umgeschaltet. Dadurch wird erreicht, daß die Meßfrequenz im Grenzbereich von MI bzw. M2 nicht ständig umgeschaltet wird.

## Patentansprüche

1. Verfahren zur Feststellung des Alkoholgehaltes und/oder des Heizwertes von Kraftstoffen durch Messung elektrisch meßbarer Größe in einer vom Kraftstoff durchströmten Meßzelle zur Bestimmung der Dielektrizitätszahl des in der Zelle enthaltenen Gemisches als Kenngröße des Alkoholgehaltes bzw. des Heizwertes mittels einer schwingfähigen Schaltung, deren Meßfrequenz derart durch Veränderung der Kapazität einstellbar bzw. anpaßbar ist, daß der Einfluß von Stoffwerten auf das Meßergebnis, welche die Kapazitätsmessunf beeinträchtigen, verringert wird.

2. Verfahren nach Anspruch 1, dadurch gekennzeichnet, daß für den Meßvorgang eine Meßfrequenz als Sollwert vorgegeben und mit dem sich aus der Messung ergebenden Istwert der sich aufgrund des tatsächlichen Alkoholgehaltes einstellenden Frequenz verglichen wird und die Frequenzdifferenz als Maß für den tatsächlichen Alkoholgehalt ausgewertet wird.

3. Verfahren nach einem der Ansprüche 1 oder 2, dadurch gekennzeichnet, daß wenigstens ein Kondensator, dessen Kapazität bekannt ist, der zu messenden Kapazität zu- bzw. von ihr abgeschaltet wird.

4. Verfahren nach einem der Ansprüche 1 bis 3, dadurch gekennzeichnet, daß die Meßfrequenz dadurch eingestellt bzw. angepaßt wird, daß eine Umschaltung zwischen Meßelektroden unterschiedlicher Kapazitätswerte erfolgt.

5. Verfahren nach einem der Ansprüche 1 bis 4, dadurch gekennzeichnet, daß die Meßfrequenz in wenigstens zwei Stufen derart umgeschaltet wird, daß sie in einem für den Einfluß die Kapazitätsmessung störender Größen unempfindlichen Raum gehalten wird.

6. Verfahren nach einem der Ansprüche 1 bis 5, dadurch gekennzeichnet, daß zusätzlich die Temperatur gemessen und zur Korrektur des festgestellten Kapazitätswertes verwendet wird.

7. Verfahren nach einem der Ansprüche 1 bis 6, dadurch gekennzeichnet, daß der festgestellte Kapazitätswert durch Kombination mit einer schaltungstechnischen und/oder rechnerischen Kompensation korrigiert wird.

8. Verfahren nach einem der Ansprüche 1 bis 7, dadurch gekennzeichnet, daß bei Anwendung auf die Steuerung bzw. Regelung einer Einspritzbrennkraftmaschine die Messung des Alkoholgehaltes bzw. des Heizwertes des zugeführten Kraftstoffs zur Vorsteuerung der Einspritzmenge dient, während die Feinregelung des Luftverhältnisses über eine Lambdaregelung bekannter Art erfolgt.

## Claims

1. A method for determining the alcohol content and/or the calorific value of fuels by measuring electrically measurable variables in a measuring cell through which the fuel flows for determining the relative permittivity of the mixture contained in the cell as a characteristic of the alcohol content or the calorific value by means of an oscillatory circuit, the measuring frequency of which can be set or adapted by changing the capacitance such that the influence of material values on the measured result, which would adversely affect the capacitance measurement, is reduced.

2. A method according to Claim 1, characterised in that for the measuring operation a measuring frequency is set as the desired value and is compared with the actual value, yielded from the measurement, of the frequency which occurs as a result of the actual alcohol content, and the frequency difference is evaluated as a measurement of the actual alcohol content.

3. A method according to one of Claims 1 or 2, characterised in that at least one capacitor, the capacitance of which is known, is switched on or switched off by the capacitance to be measured.

4. A method according to one of Claims 1 to 3, characterised in that the measuring frequency is set or adapted in that switching-over takes place between measuring electrodes of different capacitances.

5. A method according to one of claims 1 to 4, characterised in that the measuring frequency is switched in at least two stages, such that it is kept within a range which is not sensitive to the influence of variables which would disrupt the capacitance measurement.

6. A method according to one of Claims 1 to 5, characterised in that additionally the temperature is measured and is used for correcting the capacitance determined.

7. A method according to one of Claims 1 to 6, characterised in that the capacitance determined is corrected by combination with a circuitry and/or computational compensation.

8. A method according to one of Claims 1 to 7, characterised in that for application to the control or regulation of an injection internal combustion engine, the measurement of the alcohol content or of the calorific value of the fuel supplied serves for preliminary control of the injection quantity, whereas the fine control of the air ratio takes place by means of a lambda control of known type.

## Revendications

1. Méthode de détermination de la teneur en alcool et/ou de la valeur calorifique de carburants par la mesure de données mesurables électriquement dans une cellule de mesure traversée par le carburant, afin de déterminer, comme donnée caractéristique de la teneur en alcool ou de la valeur calorifique, le coefficient diélectrique du mélange contenu dans la cellule, à l'aide d'un circuit oscillant dont la fréquence de mesure est réglable ou adaptable par modification de la capacité, de telle manière que l'influence sur le résultat de mesure des valeurs de matières qui limitent la mesure de capacité soit réduite.

2. Méthode selon la revendication 1, caractérisée en ce qu'une fréquence de mesure est préétabli comme valeur de consigne pour l'opération de mesure à laquelle est comparée la valeur réelle, résultant de la mesure, de la fréquence qui s'établit sur base de la teneur en alcool effective et que la différence de fréquence est exploitée comme mesure de la teneur en alcool effective.

3. Méthode selon l'une des revendications 1 ou 2, caractérisée en ce qu'au moins un condensateur, dont la capacité est connue, est connecté à ou déconnecté de la capacité à mesurer.

4. Méthode selon l'une des revendications 1 à 3, caractérisée en ce que la fréquence de mesure est réglée ou adaptée par le fait qu'il se produit une commutation entre des électrodes de mesure à valeurs de capacités différentes.

5. Méthode selon l'une des revendications 1 à 4, caractérisée en ce que la fréquence de mesure est commutée, en au moins deux phases, de telle manière qu'elle est maintenue dans une plage insensible à l'influence de données gênant la mesure de la capacité.

6. Méthode selon l'une des revendications 1 à 5, caractérisée en ce que la température est en outre mesurée et utilisée pour la correction de la valeur de capacité déterminée.

7. Méthode selon l'une des revendications 1 à 6, caractérisée en ce que la valeur de capacité déterminée est corrigée en combinaison avec une compensation du point de vue technique des circuits et/ou par ordinateur.

8. Méthode selon l'une des revendications 1 à 7, caractérisée en ce qu'en cas d'application à la commande ou à la régulation d'une machine à injection de carburant, la mesure de la teneur en alcool ou de la valeur calorifique du carburant alimenté sert au préréglage de la quantité à injecter, tandis que le réglage fin du rapport d'air se fait par une régulation Lambda de type connu.
